# EUROPEAN PATENT APPLICATION

(11) **EP 4 501 336 A1**
(43) Date of publication of application: **05.02.2025**
(21) Application number: 23775297.7
(22) Date of filing: 22.03.2023
(51) Int. Cl.: A61K 35/17, A61P 25/28, C12N 5/0783, A61K 38/21, A61K 38/19

(54) **PHARMACEUTICAL COMPOSITION FOR PREVENTING OR TREATING DEGENERATIVE BRAIN DISEASE COMPRISING NATURAL KILLER CELL**

(30) Priority: 24.03.2022 KR 20220036570
(71) Applicant: Therabest Co., Ltd., Seoul 06656 (KR)
(72) Inventor: HWANG, Do Won, Suwon-si Gyeonggi-do 16700 (KR); KI, Young Wook, Yongin-si Gyeonggi-do 16847 (KR)
(74) Representative: HGF
(86) International application number: PCT/KR2023/003782
(87) International publication number: WO 2023/182801

(57) **Abstract**

The present invention relates to a pharmaceutical composition for preventing and treating a degenerative brain disease, specifically dementia, comprising an activated natural killer cell as an active ingredient. The pharmaceutical composition comprising a mouse-derived activated natural killer cell as an active ingredient, according to the present invention, regulates the activity of microglial cells and inhibits the deposition of amyloid β plaques. In addition, the pharmaceutical composition showed an excellent effect on cognitive function improvement in an animal model of dementia. Furthermore, when a human peripheral blood mononuclear cell-derived natural killer cell and a human induced pluripotent stem cell-derived natural killer cell were activated, it was confirmed that the expression of some genes involved in restoring the activity of microglial cells was similar to or higher than that of a mouse-derived activated natural killer cell. Therefore, the pharmaceutical composition of the present invention can be effectively used for preventing or treating a degenerative brain disease, such as dementia, Parkinson's disease, and Huntington's disease, and improving cognitive impairment.

## Description

### Technical Field

The present disclosure relates to a pharmaceutical composition for preventing or treating a degenerative brain disease comprising an activated natural killer cell as an active ingredient.

### Background Art

The immune system plays a role in protecting the body from harmful substances. Harmful substances to which the immune system reacts include bacteria, viruses, toxins, cancer cells or the blood and tissue of other organisms.

Recently, a number of methods for modulating the activity of the immune system have been studied as a method for treating various diseases in which the immune system is known to be involved. Although methods using cells consisting of the immune system are also being studied as another method of treating diseases, their use is mostly limited only to cancer treatment.

Meanwhile, dementia is a clinical syndrome in which cognitive functions in various areas, such as memory, language, and judgment, are reduced due to various acquired causes, making it difficult to carry out daily activities. Dementia patients experience symptoms of insomnia, memory impairment, decreased mobility, depression, loss of motivation, and the like.

Dementia is classified into Alzheimer's disease, vascular dementia, dementia with Lewy Body, frontotemporal dementia, and so on depending on the cause. Alzheimer's disease is characterized by progressive memory impairment, cortical amyloid plaques, and nerve fiber tangles, and accounts for 50-75% of dementia patients. Vascular dementia is a cerebrovascular disease that progresses in stages, in which memory impairment is low and concentration declines, and is characterized by spreading to single infarcts or multiple infarcts in major areas (Breijyeh et al. Molecules, 25:5789 (2020)).

Although the average life expectancy is increasing due to medical advances, a definitive treatment for dementia has not yet been developed. Thus, there is a need for research on methods that can more effectively prevent or treat dementia.

### Detailed Description of Invention

### Technical Problem

Thus, the present inventors have conducted research on effectively treating and preventing degenerative brain diseases, in particular, dementia, and have found that natural killer cells activated in vitro inhibit the deposition of amyloid β (Aβ) plaques in the brain of an animal model of dementia, and have a significantly improved effect in a cognitive function and behavioral test. In addition, gene sequence analysis showed that administration of natural killer cells having immunomodulatory functions enhances the immunometabolic and phagocytic functions of abnormal microglia present in dementia brain. As a result, the present disclosure was completed by confirming that natural killer cells can be used as a cell therapeutic agent to treat a degenerative brain disease.

### Solution to Problem

In order to achieve the above object, in one aspect of the present disclosure, there is provided a pharmaceutical composition for preventing or treating a degenerative brain disease comprising natural killer cells as an active ingredient.

In another aspect of the present disclosure, there is provided a use of the pharmaceutical composition for preventing or treating a degenerative brain disease.

In yet another aspect of the present disclosure, there is provided a method for treating a degenerative brain disease comprising administering the pharmaceutical composition to an individual.

### Effects of Invention

A pharmaceutical composition according to the present disclosure comprising mouse-derived activated natural killer cells as an active ingredient modulated the activity of microglia and inhibited the deposition of amyloid beta plaques. In addition, an excellent effect was shown in improving cognitive functions in an animal model of dementia. Furthermore, when natural killer cells derived from human peripheral blood mononuclear cells and natural killer cells derived from human induced pluripotent stem cells were activated, the expression of GM-CSF, IFNG, GADD45G, HAVCR2, HNGB1 and TNEN119 genes involved in recovering the activity of microglia were shown to be similar to or higher than that of mouse-derived activated natural killer cells. Therefore, the pharmaceutical composition of the present disclosure can be effectively used to prevent or treat degenerative brain diseases, such as dementia, Parkinson's disease, and Huntington's disease, and to improve cognitive impairment.

### Brief Description of Drawings

Figure 1 is a graph showing a culture schedule for activating mouse-derived natural killer cells.
Figures 2a and 2b are figures (top) and a graph (bottom) showing the results confirmed through FACS analysis on the surface marker of mouse-derived natural killer cells immediately after isolation (day 0) or after being activated for 7 days.
Figure 3 is a graph showing the result of observing the morphological appearance of mouse-derived natural killer cells activated for 4 days or 7 days.
Figure 4 is a graph showing the result of measuring the concentration of IFN-gamma and GM-CSF secreted from mouse-derived natural killer cells immediately after isolation (day 0) or after being activated for 7 days.
Figures 5a and 5b are graphs showing the results confirming the gene expression of mouse-derived natural killer cells immediately after isolation (day 0) or after being activated for 7 days.
Figure 6 is a graph showing the animal experiment schedule to confirm the effect of mouse-derived natural killer cells (imNK cells, immunomodulatory NK cells) activated for 7 days in improving dementia. Normal: normal mice (wild-type mice), tg: animal mouse model of dementia.
Figure 7 is a graph showing the result confirming the effect of inhibiting the formation of amyloid beta (Aβ) plaques by administering imNK cells in an animal mouse model of dementia.
Figure 8 is a graph showing the result confirming the effect of inhibiting the activity of microglia by administering imNK cells in an animal mouse model of dementia.
Figure 9 is a graph showing the result confirming the effect of improving cognitive functions and behavior through a Y-maze test after administering imNK cells in an animal mouse model of dementia.
Figure 10 is a graph showing the result of comparatively analyzing the transcriptome of microglia in an animal mouse model of dementia and normal mice (top), and the transcriptome of microglia in a group of an animal mouse model of dementia administered with imNK cells and a dementia control group (bottom).
Figure 11 is a graph showing the heatmap of genes whose expression had decreased in the control group of an animal mouse model of dementia compared to normal mice, but whose expression had increased and was restored by the administration of imNK cells.
Figure 12 is a graph showing the heatmap of genes whose expression had increased in the control group of an animal mouse model of dementia compared to normal mice, but whose expression had reduced by the administration of imNK cells.
Figure 13 is a graph showing the result confirming the expression pattern of genes involved in metabolic processes, among the genes whose expression had decreased in the control group of an animal mouse model of dementia compared to normal mice, but whose expression had increased and was restored by the administration of imNK cells.
Figure 14 is a graph showing the result confirming that the expression of genes involved in the glycolysis and gluconeogenesis among metabolic processes had normalized.
Figure 15 is a graph showing the result confirming the expression of genes involved in the neurogenesis, among the genes whose expression had decreased in the control group of an animal mouse model of dementia compared to normal mice, but whose expression had increased by the administration of imNK cells.
Figures 16a to 16d are graphs showing the results confirming the expression of genes involved in the immune response, among the genes whose expression had decreased in the control group of an animal mouse model of dementia compared to normal mice, but whose expression had increased by the administration of imNK cells.
Figure 17 is a graph showing the result confirming the expression of genes involved in the autophagy, among the genes whose expression had decreased in the control group of an animal mouse model of dementia compared to normal mice, but whose expression had increased by the administration of imNK cells.
Figure 18 is a graph showing the result confirming the expression of genes involved in the lysosomal acidification, among the genes whose expression had decreased in the control group of an animal mouse model of dementia compared to normal mice, but whose expression had increased by the administration of imNK cells.
Figure 19 is a graph showing the result confirming the expression of the lysosomal hydrolase genes, among whose expression had decreased in the control group of an animal mouse model of dementia compared to normal mice, but whose expression had increased by the administration of imNK cells.
Figures 20a to 20f are graphs showing the results of the expression of genes associated with the effector function and immune cell activation, based on the transcriptome analysis results of natural killer cells (activated PBNK) derived from human peripheral blood mononuclear cells immediately after isolation from the human peripheral blood mononuclear cells (day 0, inactive, PB-NK) and after being activated for 14 days (day 14).
Figures 21a to 21c are graphs showing the results of analyzing the expression of genes with similar or increased expression in EiNK, based on the transcriptome analysis results of natural killer cells derived from human peripheral blood mononuclear cells activated for 14 days and natural killer cells derived from human induced pluripotent stem cells activated for 21 days (EiNK, enhanced induced pluripotent stem cell derived natural killer cell).
Figure 22 is a graph showing the q-PCR result confirming the gene expressions of GM-CSF, IFNG, GADD45G, HAVCR2, HNGB1 and TNEN119 in inactivated natural killer cells derived from human peripheral blood mononuclear cells (PBNK), activated natural killer cells derived from human peripheral blood mononuclear cells (activated PBNK, 14 days) and activated natural killer cells derived from human induced pluripotent stem cells (EiNK, 21 days).

### Best Mode for Carrying out the Invention

In one aspect of the present disclosure, there is provided a pharmaceutical composition for preventing or treating a degenerative brain disease comprising natural killer cells as an active ingredient.

As used herein, the term "natural killer cells (NK cells)" refers to cytotoxic lymphocytes that constitute a major component of the innate immune system, defined as large granular lymphocytes (LGL), and constitute a third cell differentiated from B and T lymphocytes generated from a common lymphoid progenitor (CLP). As lymphoid lineage cells that play a part in the immune response, approximately 10-15% exist in the blood of a normal person and have a high killing ability when responding to non-self. Natural killer cells can non-specifically and immediately respond to cells infected with various viruses or bacterial infiltration, or the generation of abnormal cells to remove foreign substances.

Most natural killer cells existing in the body of a normal person exist in an inactive state and are activated by a response to interferon or macrophage-derived cytokines.

The natural killer cells may be obtained from an individual and obtained from various tissues. In addition, the natural killer cells may include unaltered natural killer cells obtained from an individual and may include immature natural killer cells in addition to mature natural killer cells.

Specifically, the natural killer cells may be obtained by isolating them from tissues, hematopoietic cells, hematopoietic stem cells, or hematopoietic progenitor cells. More specifically, the natural killer cells may be produced from hematopoietic cells, hematopoietic stems or progenitors, placental or umbilical cord-derived stem cells, induced pluripotent stem cells from, for example, placental tissue, placental perfusate, umbilical cord blood, placental blood, peripheral blood, spleen, liver, and the like, or cells differentiated therefrom, but are not limited thereto. The natural killer cells may be cells obtained from autologous or allogeneic sources. In addition, the natural killer cells may be derived from mammals, such as rats, mice, livestock, humans, and the like. In one embodiment, they may be obtained from an individual seeking to be treated with natural killer cells. In such case, the natural killer cells may be directly isolated from the blood of the individual for use or can be used by differentiating immature natural killer cells or stem cells obtained from the individual.

The natural killer cells of the present disclosure may be obtained from blood or differentiated from stem cells or induced pluripotent stem cells.

As used herein, the term "stem cells" refers to relatively underdeveloped, undifferentiated cells that have the ability to differentiate into specific tissue cells under suitable conditions. The stem cells may self-generate to create daughter cells with the ability to maintain their own undifferentiated characteristics, and simultaneously create daughter cells that can differentiate into a specific type of cells. The stem cells include embryonic stem cells, adult stem cells, and induced pluripotent stem cells.

As used herein, the term "induced pluripotent stem cells (iPSC)" refers to cells generated to have pluripotency like embryonic stem cells by inducing dedifferentiation in non-pluripotent somatic cells. The induced pluripotent stem cells may also be called reprogrammed stem cells. The induced pluripotent stem cells may be generated using the somatic cells of a fetus, a newborn, a child, or an adult. The induced pluripotent stem cells may be derived from fibroblasts, keratinocytes, blood cells, or kidney epidermal cells. The induced pluripotent stem cells may be produced by reprogramming somatic cells. Reprogramming somatic cells to manufacture into induced pluripotent stem cells may use a known method, for example, the method disclosed in Takahashi K, Yamanaka S (August 2006), Cell, Vol. 126, No. 4, pp. 663-676. The induced pluripotent stem cells may be differentiated into all cells constituting one or more types of tissues or organs, or preferably any one of three germ layers: endoderm (inner stomach lining, gastrointestinal tract, and lung), mesoderm (muscle, bone, blood, and the genitourinary system) or ectoderm (epidermal tissue and nervous system). As used herein, the natural killer cells differentiated from iPSCs are referred to as "EiNK."

In the present disclosure, the natural killer cells are activated as a response to interferon or macrophage-derived cytokines, and the natural killer cells include two types of extracellular receptors that control the cytotoxic activity of cells labeled "activating receptor" and "inhibitory receptor."

In the present disclosure, the natural killer cells may be an overexpression of any one specific gene selected from the group consisting of GM-CSF, IFN-gamma, Gadd45g, zbtb32, P2RY14, TREM119, IL12RB1, CD55, Sema6D, Gzmb/c/d/e/f, Havcr2, CXCR6, HMGB1, IL-2, IL-10 and combinations thereof.

As used herein, the term "GM-CSF (granulocyte-macrophage colony-stimulating factors)" refers to a protein secreted by macrophage, T cells, mast cells, natural killer cells, endothelial cells, and fibroblasts. As a cytokine that functions as a growth factor of white blood cells, it regulates the differentiation and proliferation of bone marrow progenitor cells and plays a role in enhancing the function of mature monocytes and granulocytes.

As used herein, the term "IFN-gamma" refers to a cytokine secreted by natural killer cells, Th1 cells, cytotoxic T cells, and the like, and plays an important role in the function of most immune cells and in innate and adaptive immune responses. IFN-gamma is known to have various biological effects, such as anti-viral activity, inhibition of cell or tumor growth, and promotion of terminal differentiation of B cells into immunoglobulin-producing cells. In addition, it activates macrophages and increases cytotoxicity of natural killer cells and T cells.

As used herein, "Gadd45g (growth arrest and DNA-damage 45-inducible gamma)", also known as CR6, DDIT2, GRP17 or OIG37, regulates sexual development, human-specific brain development, tumor suppression, cellular stress response, and the like. It is known that it activates MAP kinase in helper T cells (in particular, Th1 cells) and is involved in IFN-gamma production and anti-tumor immune response along with Gadd45b.

As used herein, the term "zbtb32 (zinc finger and BTB domain containing 32)" refers to a transcriptional repressor mainly expressed in activated T cells and B cells. The expression of zbtb32 is known to be induced by inflammatory cytokines to promote the proliferation of natural killer cells. In addition, it suppresses the expression of the CIITA (class II major histocompatibility complex transactivator) gene to regulate an immune response.

As used herein, the term "P2RY14 (P2Y purinoceptor 14)" refers to a receptor belonging to the G-protein coupled receptor (GPCR) family. It is known that it plays an important role in in the functional homeostasis of microglia and is involved in anti-inflammation by reducing IL-6. It is known that it suppresses cellular senescence in stem cells and progenitor cells, and the selective high-affinity antagonist against P2RY14 suppresses the UDP-glucose-stimulated chemotaxis of human neutrophils.

As used herein, the term "TREM119 (transmembrane protein 119)" refers to a surface protein that plays a key role in the functional homeostasis of microglia present in the brain. It is also known as a protein that plays an important role in bone formation by promoting the differentiation of myoblasts into osteoblasts.

As used herein, the term "IL12RB1 (interleukin-12 receptor subunit beta-1)" refers to an interleukin receptor, which alone binds to IL-12 with low affinity. It binds to IL-12RB2 to form a high-affinity receptor for IL-12, and when it binds to IL-23R, it acts as a receptor for IL-23.

As used herein, "CD55", also known as a complement decay-accelerating factor or DAF, is a protein that is involved in the complement action on the cell surface.

As used herein, the term "Sema6D (semaphoring-6D)" refers to a protein expressed in T cells, B cells and natural killer cells, and is known to bind to plexin-A1 expressed in dendritic cells (DC). Sema6D activates dendritic cells by binding to plexin-A1 of the dendritic cells in T cells, promotes the production of type 1 interferon in B cells, and regulates the activity of T cells during the primary immune response. In addition, it is known to be involved in differentiating natural killer cells into osteoclasts.

As used herein, the term "Gzm (granzyme)" refers to a serine protease released by cytoplasmic granules in cytotoxic T cells and natural killer cells. The granzyme is packed into cytotoxic granules along with perforin and secreted into immune-targeted cells to induce the apoptosis of target cells. In humans, granzymes A, B, H, K and M are expressed, among which it is known that granzymes A and B are expressed the most. In addition, the granzyme plays a role in removing intracellular pathogens. Granzyme B induces the apoptosis of target cells in caspase-dependent and independent manners.

As used herein, "Havcr2 (hepatitis A virus cellular receptor 2)", also known as TIM-3 (T-cell immunoglobulin and mucin-domain containing-3), is expressed on the surface of T cells, monocytes, macrophages, dendritic cells, mast cells, natural killer cells and cancer cells. As an immune checkpoint protein, it suppresses the activity of immune cells by binding to Gal-9, PtdSer, HMGB1, and CEACAM1.

As used herein, the term "HMGB1 (high mobility group box 1)" refers to a chromatin component protein that binds to nucleosomes, transcription factors, and histones in the nucleus to regulate transcription by inducing chromatin remodeling. In addition, HMGB 1 is secreted from dead cells or immune cells, such as monocytes, macrophages, and dendritic cells, to induce an inflammatory response. HMGB1 secreted from cells is known to activate an inflammatory response by binding to TLR2 (toll-like receptor 2) and TLR4 to increase cytokine secretion from macrophages. In contrast, when it binds to the immune checkpoint protein, TIM-3, an immune response is suppressed by suppressing the TLR signaling.

As used herein, "IL-2", also known as a T-cell growth factor (TCGF), is a spherical glycoprotein that plays a central role in lymphocyte production, survival, and homeostasis. IL-2 is mainly produced by activated T cells, in particular, helper T cells. IL-2 stimulates the proliferation and differentiation of T cells and induces the generation of cytotoxic T cells and the differentiation of peripheral blood lymphocytes into cytotoxic cells and lymphokine-activated killer cells (LAK cells).

As used herein, "IL-10", also known as CSIF (human cytokine synthesis inhibitory factor), is an anti-inflammatory cytokine. It is mainly produced by monocytes, Th2 cells, mast cells, regulatory T cells, and activated B cells. IL-10 suppresses an immune response by suppressing NF-κB activity and the JAK-STAT signaling pathway. In addition, it regulates the proliferation and differentiation of various immune cells, such as T cells, B cells, natural killer cells, antigen presenting cells (APC), mast cells, and granulocytes.

In the present disclosure, the gene may be overexpressed by two times or more compared to natural killer cells obtained from an individual.

As used herein, the term "natural killer cells obtained from an individual" refers to cells of the natural killer cell lineage that have not yet developed into mature natural killer cells, as indicated by the level of expression of one or more marker proteins, for example, CD56, CD16, and KIR. Specifically, they may be natural killer cells derived from the individual that have no additional modifications and have not induced their own activation, or natural killer cells on day 0 of culture.

Specifically, the natural killer cells according to the present disclosure may have an overexpression of the gene by about 2 times or more, about 3 times or more, about 4 times or more, about 5 times or more or about 6 times or more compared to natural killer cells obtained from an individual.

The natural killer cells according to the present disclosure may be activated natural killer cells.

As used herein, the term "activated natural killer cells" may refer to cells having increased cytotoxicity or having enhanced immune-modulating abilities inherent to the natural killer cells, compared to natural killer cells that have been induced to differentiate into NK cells from hematopoietic cells, natural killer progenitor cells, iPSCs or stem cells or derived from any tissue and that have no additional modifications. The activated natural killer cells may be cells in which the receptor that can target the cells or tissue to be treated is overexpressed. Whether the natural killer cells are activated can be evaluated by detecting one or more types of functionally related markers, for example, CD161, NKp44, DNAM-1, 2B4, NKp46, CD107a, and the NKG2 family of activation receptors (for example, NKG2D).

Methods for activating natural killer cells may include culturing natural killer cells derived from an individual under specific conditions, genetically modifying them, or treating them with substances that increase the activity of natural killer cells.

In the present disclosure, the natural killer cells may be those cultured in a medium and included in the culture medium, or those cultured in a medium and then isolated from the culture medium.

To culture natural killer cells, conventional animal cell culture media known in the art may be used without limitation, for example, CellGro medium (Cellgenix), AIM-V medium, RPMI1640 medium, XVIVO 20, or RPMI1640, may be used.

As used herein, the term "substance that increases the activity of natural killer cells" refers to any substance that may increase the activity of natural killer cells, for example, the cytotoxicity of natural killer cells, or may produce, increase, or proliferate activated natural killer cells.

A substance that increases the activity of natural killer cells may include immune cytokines, agonists for natural killer cell activation receptors, or antagonists against natural killer cell inhibitory receptors, but is not limited thereto.

As used herein, the term "cytokine" refers to a biostimulatory signaling substance that controls the defense system in the body, and may refer to a protein that regulates physiological activity. A cytokine regulates various biological activities, such as cell activation, differentiation, cell migration, senescence, and death induction, in various cells through autocrine and paracrine actions.

A cytokine may be in the interferon (IFN) family, the interleukin family, the tumor necrosis factors (TNF) family, or combinations thereof.

Interferon may be any one selected from the group consisting of type 1 interferon (e.g., interferon-α, interferon-β, interferon-x, interferon-ω), type 2 interferon (e.g., interferon-γ), type 3 interferon, and combinations thereof. Interleukin may be any one selected from the group consisting of IL-2, IL-5, IL-8 IL-12, IL-15, IL-18, IL-21, and combinations thereof. TNF may include TNF-α, TNF-β, or TNF-γ. An immune cytokine may include a cytokine-antibody complex. For example, it may include an interleukin-anti-interleukin antibody complex.

In one embodiment, the natural killer cells of the present disclosure may be obtained from an individual or by inducing a differentiation of iPSC cells into NK cells and then culturing the cells in a medium in which IL-15 is present. More specifically, the natural killer cells may be obtained by culturing the natural killer cells obtained from the spleen or peripheral blood in a medium containing IL-15 for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 22 days, 23 days, 24 days, 25 days, 26 days, 27 days, or 28 days. The natural killer cells may be obtained by culturing the natural killer cells differentiated from iPSCs in a medium containing IL-15 for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 22 days, 23 days, 24 days, 25 days, 26 days, 27 days, or 28 days. The concentration of IL-15 contained in the medium may be 10 ng/ml to 200 ng/ml.

In one embodiment, the natural killer cells of the present disclosure may be obtained from an individual or obtained by inducing a differentiation of iPSC cells into NK cells and then culturing the cells in a medium in which IL-2 is present. More specifically, the natural killer cells may be obtained by culturing the natural killer cells obtained from the spleen or peripheral blood in a medium containing IL-2 for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 22 days, 23 days, 24 days, 25 days, 26 days, 27 days, or 28 days. The natural killer cells may be obtained by culturing the natural killer cells differentiated from iPSCs in a medium containing IL-2 for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 22 days, 23 days, 24 days, 25 days, 26 days, 27 days, or 28 days. The concentration of IL-2 contained in the medium may be 20 IU/ml to 200 IU/ml.

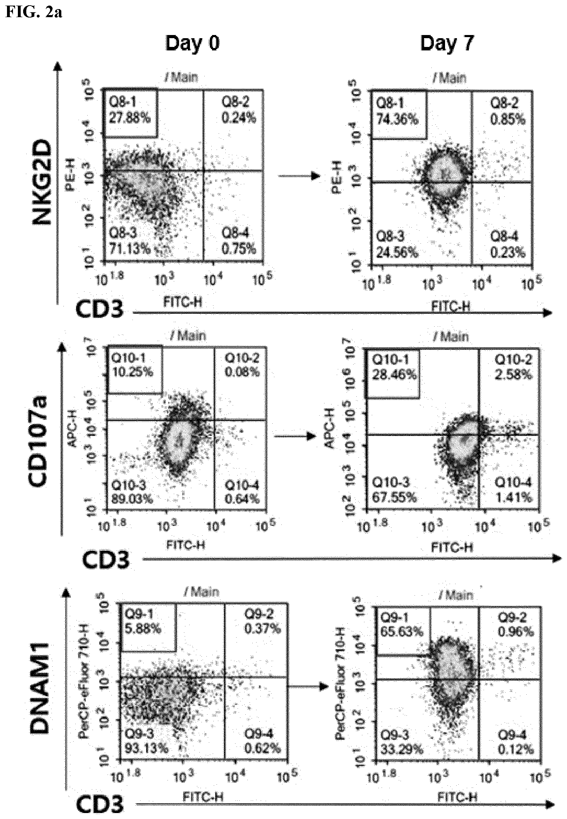

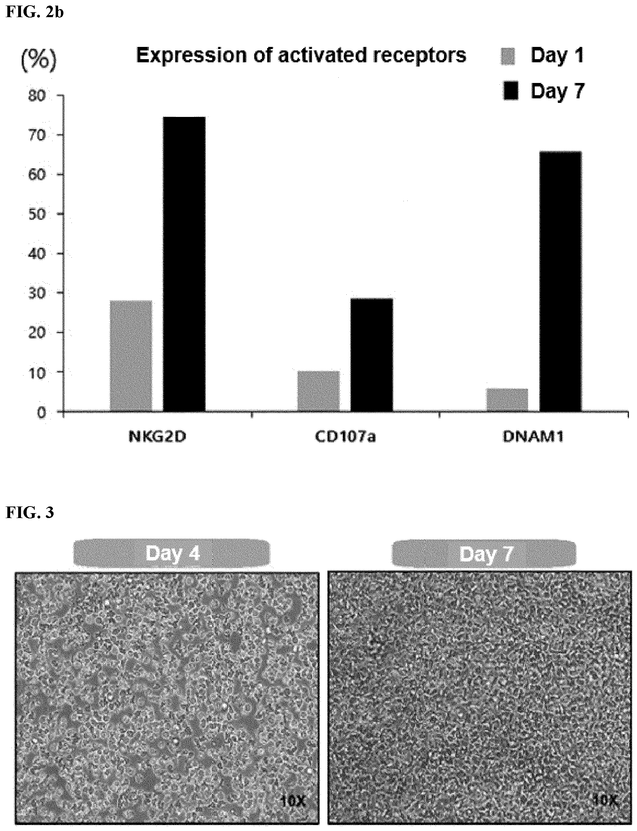

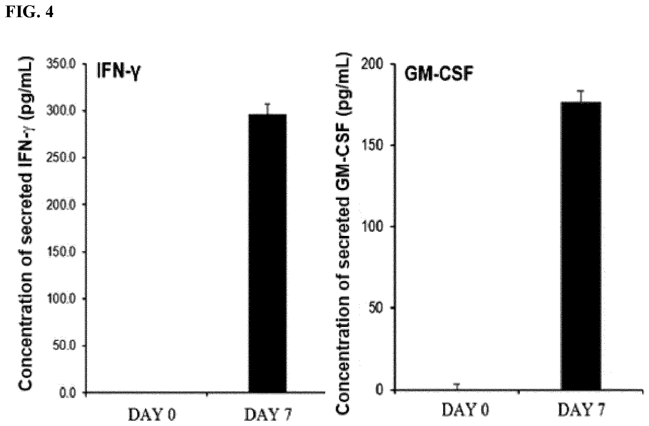

In one embodiment of the present disclosure, natural killer cells isolated from the spleen activated by IL-15 for 7 days exhibit the phenotypes of CD3-NKG2D+, CD3-DNAM-1+, and CD3-CD107a+ (Figure 2), and the secretion of INF-gamma and GM-CSF increased by 150 times or more compared to day 0 of IL-15 treatment (Figure 4).

A pharmaceutical composition comprising the natural killer cells of the present disclosure may induce the removal of amyloid β (Aβ).

It is known that when "amyloid beta" as used herein is accumulated and agglomerated in the brain to form plaques, it can be toxic and destroy synapses, which are signaling systems of nerve cells, thereby causing dementia symptoms. Therefore, the pharmaceutical composition according to the present disclosure may have an effect of preventing or treating dementia by inducing the removal of amyloid beta plaques.

The pharmaceutical composition according to the present disclosure may regulate the activity of microglia.

As used herein, the term "microglia" refers to a form of neuroglial cells that are distributed throughout the brain and the spinal cord and account for 5-12% of cells in the central nervous system. They are immune cells that reside in the brain and are closely involved in brain homeostasis, host defense against pathogens, and the like. Until recently, microglia cells are defined as innate immune cells of myeloid origin that express CX3CR1, CD11b, Iba1, and f4/80. Microglia play an important role in various developmental stages from the embryonic stage to adulthood and aging. In particular, they form a network throughout the central nervous system thereby maintaining homeostasis and performing a sensing function for host defense functions, and play an important role in synaptic remodeling (important for the development, homeostasis, and neurodegeneration of the central nervous system) and maintaining myelin homeostasis. In addition, they are involved in homeostasis maintenance, inflammation, and neurodegeneration through an interaction with astrocytes.

As used herein, the term "degenerative brain disease" refers to a disease of the brain among degenerative diseases occurring with age. It is known to occur when specific cell groups in the brain and the spinal cord gradually lose their function due to as yet unknown causes, by the death of brain nerve cells, which are the most important cells in transmitting information in the brain nervous system, by a problem in the formation or function of synapses transmitting information between nerve cells in the brain, and by an abnormal increase or decrease in the electrical activity of brain nerves.

The degenerative brain disease may be any one selected from the group consisting of Alzheimer's disease, Parkinson's disease, Huntington's disease, mild cognitive impairment, cerebral amyloid angiopathy, Down syndrome, amyloid stroke, systemic amyloidosis, amyloidosis-Dutch type, Niemann-Pick disease, senile dementia, amyotrophic lateral sclerosis, spinocerebellar atrophy, Tourette's syndrome, Friedrich's ataxia, Machado-Joseph disease, Lewy body dementia, vascular dementia, dystonia, progressive supranuclear palsy, and frontotemporal dementia, but is not limited thereto.

As used herein, the term "prevent" refers to all actions that suppress or delay the onset of geriatric diseases by administering the pharmaceutical composition. The term "treat" refers to all actions that improve or beneficially change the symptoms of a degenerative brain disease by administering the pharmaceutical composition.

The natural killer cells may be included in any amount (effective amount) based on the use, formulation, purpose of compounding, and the like, as long as they can exhibit activity in the pharmaceutical composition of the present disclosure for preventing or treating a degenerative brain disease. The pharmaceutical composition of the present disclosure may include 1×10² to 1×10¹⁰, 1×10³ to 1×10⁹, 1×10⁴ to 1×10⁸, or 1×10⁵ to 1×10⁷ activated natural killer cells. Preferably, the pharmaceutical composition may include 2×10⁶ activated natural killer cells.

As used herein, the term "effective amount" refers to the amount of an active ingredient that can induce an anti-aging effect. This effective amount may be determined experimentally within the scope of the ordinary ability of those skilled in the art.

The composition of the present disclosure may be used unfrozen or frozen for later use. If it needs to be frozen, standard cryopreservatives (e.g., DMSO, glycerol, polyvinylpyrrolidone, polyethylene glycol, albumin, dextran, sucrose, ethylene glycol, i-d erythritol, D-ribitol, D-mannitol, D-sorbitol, i-inositol, D-lactose, choline chloride, and Epilife^{®} cell freezing medium (Cascade Biologics)) may be added to the cells before freezing.

The pharmaceutical composition of the present disclosure comprising the natural killer cells as an active ingredient may be applied in the form of a cell therapy agent and may be formulated by further comprising a pharmaceutically acceptable carrier.

As used herein, the term "cell therapeutic agent" refers to a medicament used for the purposes of treatment, diagnosis, and prevention with cells and tissues manufactured through isolation, culture, and special manipulation from a subject (according to US FDA regulation), which may be used for the purposes of treatment, diagnosis, and prevention through a series of actions, such as altering the biological characteristics of cells by proliferation, selection or other methods on living autologous, allogeneic, or xenogeneic cells *in vitro* to restore the functions of cells or tissues. Cell therapeutic agents are largely classified into somatic cell therapeutic agents and stem cell therapeutic agents based on the degree of cell differentiation. In the present disclosure, the cell therapeutic agent may be a somatic cell therapeutic agent.

As used herein, the term "pharmaceutically acceptable carrier" refers to a carrier or a diluent that does not significantly irritate living organisms and does not inhibit the biological activity and properties of the administered substance. In the present disclosure, a pharmaceutically acceptable carrier that may be included in a cell therapeutic agent may include without limitation those known in the art, such as buffers, preservatives, analgesics, solubilizers, isotonic agents, stabilizers, bases, excipients, lubricants, and the like. The pharmaceutical composition of the present disclosure may be prepared according to commonly used techniques in the form of various dosage forms.

The pharmaceutical composition of the present disclosure may be administered in a pharmaceutically effective amount and may be administered through any route as long as it can induce a movement to the diseased area. As used herein, the term "administer" refers to introducing a given substance into an individual by an appropriate method. In some cases, loading the natural killer cells onto a vehicle equipped with a means of directing them to the lesion may be considered for the pharmaceutical composition of the present disclosure.

Therefore, the pharmaceutical composition of the present disclosure may be administered through various routes including topical (including buccal, sublingual, dermal, and intraocular administration), parenteral (including subcutaneous, intradermal, intramuscular, infusion, intravenous, intraarterial, intraarticular, and intracerebrospinal fluid) or transdermal administration, and preferably administered directly to the affected area. In one embodiment, the natural killer cells may be administered to an individual by means of suspension in a suitable diluent, wherein this diluent is used to protect and maintain the cells, and to facilitate their use when injected into the target tissue. The diluent may be saline solution, phosphate buffer solution, buffer solution, such as HBSS, cerebrospinal fluid, and the like. In addition, the composition may be administered by any device that allows an active substance to move to the target cell.

The preferred method of administration and formulation are injections. Injections may be prepared using aqueous solvents, such as saline solution, IV drip, Hanks' solution or sterilized solution, vegetable oils, such as olive oil, higher fatty acid esters, such as ethyl oleate, and non-aqueous solvents, such as ethanol, benzyl alcohol, propylene glycol, polyethylene glycol, or glycerin. A non-penetrating agent known in the art that is suitable for the barrier to be penetrated may be used for the penetration of mucosal barriers. Pharmaceutical carriers may be further added, including stabilizers to prevent deterioration such as ascorbic acid, sodium hydrogen sulfite, BHA, tocopherol, EDTA, and the like, emulsifiers, buffers for pH adjustment, preservatives to inhibit microbial growth such as phenylmercuric nitrate, thimerosal, benzalkonium, phenol, cresols, benzyl alcohol, and the like. The formulation of the pharmaceutical composition is known in the art, and a specific literature [Remington's Pharmaceutical Sciences (19th Ed., 1995)] and the like may be referred. The literature is considered a part of the present disclosure.

As used herein, the term "pharmaceutically effective amount" refers to an amount that is sufficient to treat a disease with a reasonable benefit/risk ratio applicable to medical treatment and does not cause side effects, and the effective dose level may easily be determined by those skilled in the art according to factors including the sex, age, weight, and health condition of the patient, type and severity of the disease, activity of the drug, sensitivity to the drug, method of administration, time of administration, administration route and excretion rate, duration of treatment, combined or simultaneously used drugs, and other factors known in the medical field.

The preferred dosage of the pharmaceutical composition of the present disclosure may be about 1×10³ cells/kg to about 1×10⁹ cells/kg or about 1×10⁴ cells/kg to about 1×10⁸ cells/kg per day depending on the condition, weight, sex, age of the patient, severity of the patient, and the administration route.

However, the dosage may be prescribed diversely depending on factors, such as the formulation method, mode of administration, age, weight, sex, pathological condition, and diet of the patient, administration time, administration route, excretion rate, and reaction sensitivity, and those skilled in the art may appropriately adjust the dosage by considering these factors. The number of administrations may be once or two or more times within a range of clinically tolerable side effects. The pharmaceutical composition of the present disclosure may be administered 1 to 3 times/day, 1 to 3 times/week or 1 to 10 times/month. For example, the pharmaceutical composition of the present disclosure may be administered 1 to 9 times/month, 1 to 8 times/month, 1 to 7 times/month, 1 to 6 times/month, 1 to 5 times/month, 1 to 4 times/month, 1 to 3 times/month or 1 to 2 times/month. Also, regarding the administration site, it may be administered at 1 or 2 or more sites. Also, for animals other than humans, the same dosage as a human per kg or per individual may be administered, or an amount converting the dosage above to, for example, the volume ratio (e.g., average value) of an organ (e.g., heart) of the intended animal to a human. This dosage should not be construed as limiting the scope of the present disclosure in any aspect.

As used herein, the term "individual" refers to a subject who has developed or may develop dementia to which the composition of the present disclosure may be applied (prescribed), and may be a mammal including humans, rats, mice, livestock, and the like. Preferably, it may be a human but is not limited thereto.

The pharmaceutical composition of the present disclosure may additionally include known substances that have an effect in preventing or treating dementia as needed.

The pharmaceutical composition according to the present disclosure may further include a substance that increases the activity of the aforementioned natural killer cells. The pharmaceutical composition of the present disclosure may be administered in combination with a conventionally known substance for preventing or treating dementia. In this case, the natural killer cells, the substance that has an effect in preventing or treating dementia and/or the substance that increases the activity of natural killer cells may be administered simultaneously or consecutively.

In another aspect of the present disclosure, there is provided a use of the pharmaceutical composition for preventing or treating a degenerative brain disease. In this case, the pharmaceutical composition, the degenerative brain disease, the prevention, and the treatment are as described above.

In yet another aspect of the present disclosure, there is provided a method for treating a degenerative brain disease comprising administering the pharmaceutical composition to an individual. In this case, the pharmaceutical composition, the degenerative brain disease, the administration, and the treatment are as described above.

The individual may be any animal including humans, rats, mice, livestock, and the like which has developed or may develop dementia. Preferably, it may be human.

The dosage may be about 1×10³ cells/kg to about 1×10⁹ cells/kg or about 1×10⁴ cells/kg to about 1×10⁸ cells/kg depending on the condition, weight, sex, and age of the patient, the severity of the disease, and the administration route. However, the dosage may be prescribed diversely depending on factors, such as the formulation method, mode of administration , age, weight, sex, pathological condition, and diet of the patient, administration time, administration route, excretion rate, and reaction sensitivity, and those skilled in the art may appropriately adjust the dosage by considering these factors.

### Mode for Carrying out the Invention

Hereinafter, the present disclosure will be described in more detail by way of the following examples. However, the following examples are only for illustrating the present disclosure, and the scope of the present disclosure is not limited only to these examples.

### Example 1. Cultures of mouse-derived natural killer cells

### Example 1.1. Isolation of mouse-derived natural killer cells

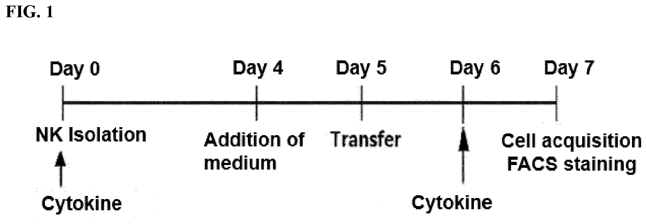

After collecting spleen cells from the spleen of a Balb/c six-week-old male mouse, an NK cell isolation kit (mouse) (Miltenyi Biotec, 130-115-818) was used to isolate natural killer cells (NK cells). The isolated natural killer cells were inoculated at 5×10⁶ cells/5 ml (T-25 flask) to be cultivated in a RPMI-1640 (Cytiva, SH30027) medium containing 10% fetal bovine serum (FBS) (Gibco, 10100147), 1% antibiotic/antibacterial agent (Gibco, 15240-062), 1% L-Glutamine (Gibco, 25030081), β-mercaptoethanol (Sigma, M3148), and a recombinant human IL-15 protein (R&D Systems, 247-ILB) (Table 1). A medium was added every day starting from day 4 of the culture and cultured for seven days to obtain mouse-derived natural killer cells (imNK cells) (Figure 1).

**[Table 1]**

| | T-25 Flask | | T-75 Flask | | T-150 Flask | |
|---|---|---|---|---|---|---|
| | Day 0 | Day 3 | Day 4 | Day 5 | Day 6 | Day 7 |
| RPMI1640 media | 5 ml | 5 ml | 10 ml | 20 ml | 40 ml | 50 ml |
| 10 % FBS | 500 µl | 500 µl | 1 ml | 2 ml | 4 ml | 5 ml |
| 1% antibiotic/antibacterial agent | 50 µl | 50 µl | 100 µl | 200 µl | 400 µl | 500 µl |
| 1 % L-glutamine | 50 µl | 50 µl | 100 µl | 200 µl | 400 µl | 500 µl |
| IL-15 | 2.5 µl | 2.5 µl | 5 µl | 10 µl | 20 µl | 25 µl |
| β-mercaptoethanol | 5 µl | 5 µl | 10 µl | 20 µl | 40 µl | 50 µl |
| Total volume | 5 ml | 10 ml | 20 ml | 40 ml | 80 ml | 130 ml |

To identify the characteristics of the imNK cells obtained using the method above, the surface marker expression (Figure 2) and the cell morphology (Figure 3) of the natural killer cells were examined.

The expression of the cell surface marker was identified by staining with anti-CD3 antibodies (Invitrogen, 11-0031-82), anti-NKG2D antibodies (Ebioscience, 25-5882-81), anti-DNAM1 antibodies (Biolegend, 128814), and anti-CD107a antibodies (BD bioscience, 560646) to perform a FACS analysis. As a result, it was identified that the imNK cells were activated natural killer cells (Figure 2). In addition, morphologically, it was identified that the morphology of the natural killer cells was maintained on day 4 of IL-15 treatment, and it was identified that the proliferation rate increased on day 7 of IL-15 treatment compared to day 4 (Figure 3).

### Example 1.2. Confirmation of cytokines secreted according to culture period of natural killer cells

During isolation and culture in the same manner as in Example 1.1, the culture medium was collected on days 0 and 7 to measure each of IFN-gamma and GM-CSF in the medium using a Mouse IFN-gamma Quantikine ELISA Kit (R&D Systems, SMIF00) and a Mouse GM-CSF Quantikine ELISA Kit (R&D Systems, MGM00). The obtained culture medium was diluted for use in ratios of stock solution, 1/10, 1/100, and 1/500.

As a result, IFN-gamma and GM-CSF were detected at 0±0.18 pg/ml and 0±3.50 pg/ml, respectively, in the culture medium of natural killer cells on day 0 of culture, confirming that almost no IFN-gamma and GM-CSF were secreted.

On the other hand, as cell culture and proliferation progressed and cell activation occurred, on day 7 when culture was completed, it was confirmed that IFN-gamma (296.16±10.87 pg/ml) and GM-CSF (176.19±7.03 pg/ml) significantly increased (Figure 4).

### Example 1.3. Confirmation of changes in gene expressions according to culture period of natural killer cells

Mouse-derived natural killer cells isolated and cultured in the same manner as in Example 1.1 were collected on days 0 and 7 of the culture to extract RNA. The extracted RNA was used to carry out mRNA sequencing using QuantSeq 3' mRNA-Seq to confirm gene expressions.

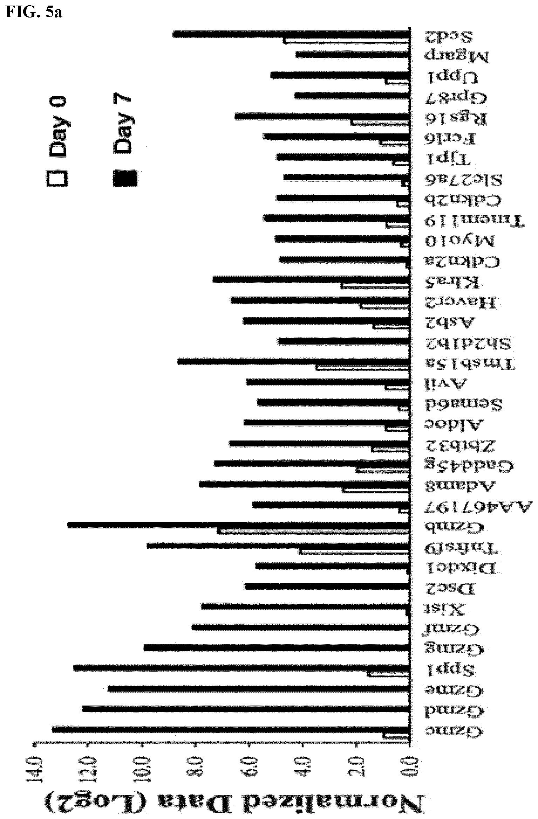

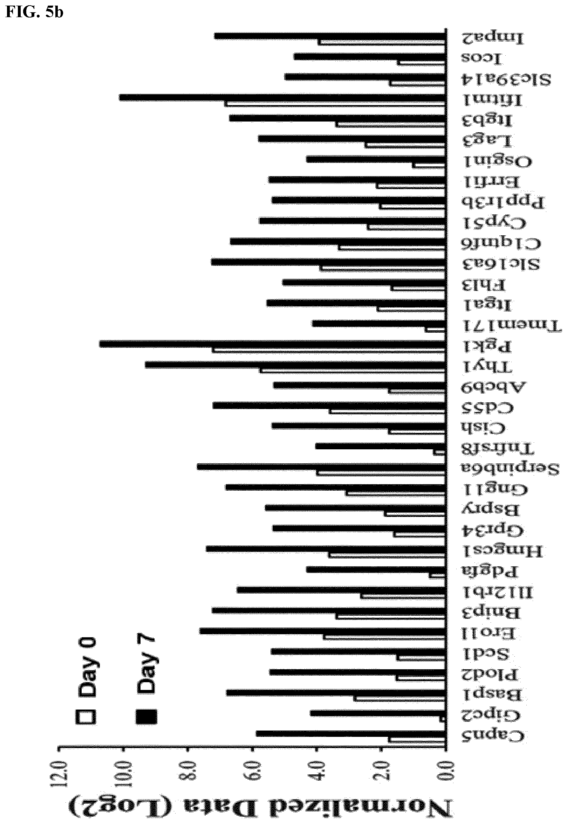

As a result, 23,183 gene expressions were confirmed in total, and it was confirmed that there were 515 genes with increased expression after completion of the culture (day 7) compared to immediately after isolation (day 0). Among these, it was confirmed that 104 genes had a fold change of 6 or more, and the 104 genes were arranged in descending order of fold change. Among the 104 genes identified, the function of several genes of interest are as described below (Figure 5).

It is known that Gadd45g activates MAP kinase in TH1 cells, participates in the production of IFN-gamma, and plays an important role in anti-tumor immune responses along with Gadd45b. Gzmc attacks nuclear and mitochondrial targets to rapidly induce target cell death, and Zbtb32 suppresses the expression of CIITA (class II major histocompatibility complex transactivator) genes to suppress the immune system. Sema6D plays a role in regulating the late-phase activity of T cells during the primary immune response, and Havcr2 and Klra2 participate in suppressing KIR signaling. In addition, TREM119 is involved in homeostasis, P2RY14 plays a role in regulating immune responses, and CXCR6 regulates the homeostasis of natural killer cells in liver tissue. HMGB1 performs the dual function of a non-histone nuclear protein and an extracellular inflammatory cytokine, and CD55 plays a role in regulating complement activation.

### Example 2. Confirmation of effect of imNK cells in preventing and treating dementia

The effect of mouse-derived natural killer cells (hereinafter referred to as imNK or imNK cells) activated in vitro as produced above in treating dementia was confirmed using a genetically modified mouse model of dementia (APP/PS1 mouse, Jackson laboratory).

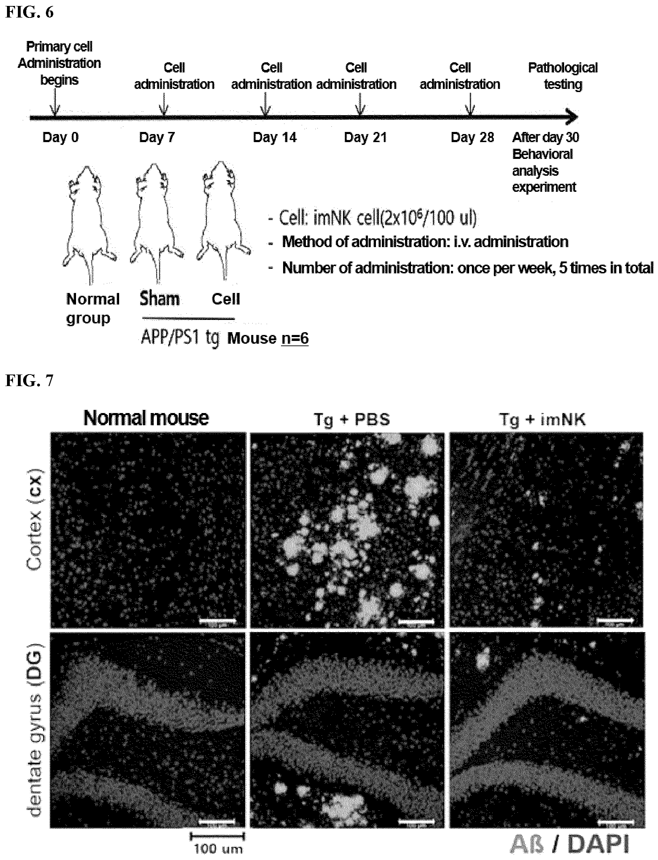

Dementia model mice were intravenously administered with imNK cells at a concentration of 2×10⁶ cells/100 µL per mouse, once every 7 days for a total of 5 times. Wild-type mice (normal group) were used as the control group against the mouse model of dementia, and the vehicle (PBS) was intravenously administered to each control group (Figure 6).

### Example 2.1. Confirmation of effect of dementia mouse model in cognitive function and behavioral improvement according to administration of imNK cells

After 30 days of administering imNK cells in the same manner as above, a Y-maze test was conducted to confirm the effect of cognitive function improvement in mice by administering imNK cells.

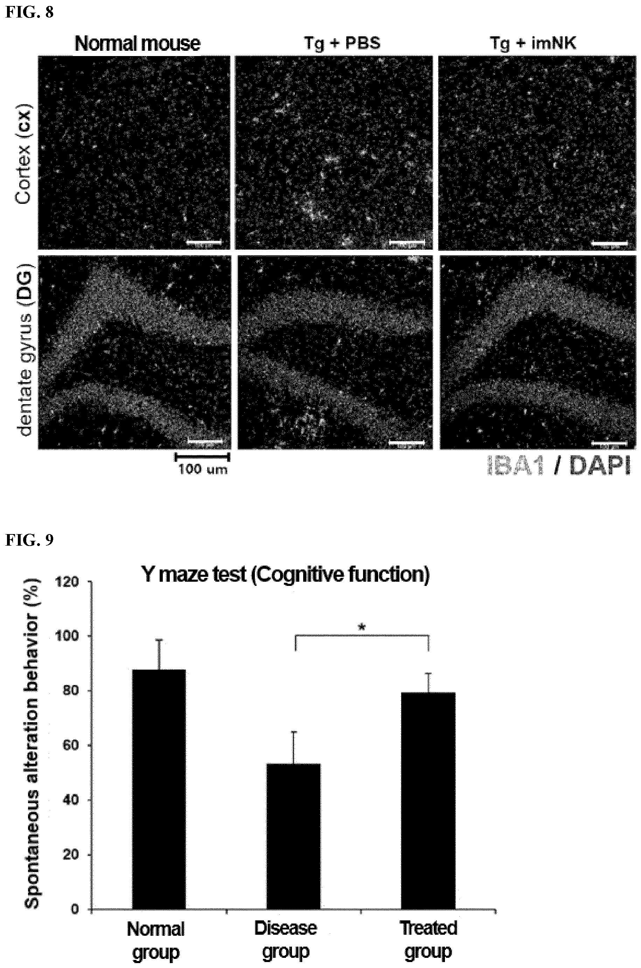

As a result, it was confirmed that the normal mouse group (normal group) instinctively found a way out while wandering around the Y-shaped maze, but the dementia mouse model group (sham, disease group) wandered around the maze less frequently. In comparison, it was confirmed that the imNK cell administered group (treated group) instinctively found a way out while wandering around the Y-shaped maze in a similar manner as the normal mouse group (normal group). These results confirmed that administration of imNK cells improved cognitive function and behavior in the mouse model of dementia (Figure 9).

### Example 2.2. Confirmation of effect of suppressing amyloid beta plaque formation in dementia mouse model according to administration of imNK cells

Amyloid β (Aβ) proteins can clump together to form plaques, which are known to have toxicity to destroy synapses, which are the signaling system of nerve cells, thereby causing dementia symptoms. Therefore, to confirm the effect of imNK cells in treating dementia, changes in amyloid beta plaque formation by the administration of imNK cells were examined.

After 40 days of administering imNK cells to the mouse model of dementia in the same manner as above, the brains of the mice were extracted, and immunostaining was carried out on the extracted brain tissue sections using an antibody that specifically binds to amyloid beta (Cell signaling technology, 8243).

The results showed an increase in amyloid beta plaque formation in the brain tissue of the mouse model of dementia (Tg+PBS) in comparison to normal mice (normal), confirming the dementia model was well established. On the other hand, in the case of the imNK cell administered group (Tg+imNK), it was confirmed that amyloid beta plaque formation was significantly reduced in the cortex area of the brain (Figure 7, read (Aβ): amyloid beta, blue (DAPI): cell nucleus). The results confirmed that when imNK cells inhibit the formation of amyloid beta plaque, thereby exhibiting an effect of preventing or treating dementia.

### Example 2.3. Confirmation of effect of inhibiting activity of microglia in dementia mouse model according to administration of imNK cells

In general, microglia, which are known as immune cells of the brain, are present in the brain, and it is reported that microglia are activated in the brains of mice with dementia. Therefore, changes in the activity of microglia by the administration of imNK cells were examined to confirm the effect of imNK cells in treating dementia.

On day 40 of administering imNK cells to the mouse model of dementia in the same manner as above, the brains of the mice were extracted, and immunostaining was carried out on the extracted brain tissue sections using an antibody that specifically binds to IBA1 (Abcam 153696), which is a microglial cell marker.

The results showed that the expression of IBA1 increased in the dentate gyrus and cortex in the brains of the mouse model of dementia (Tg+PBS), confirming that microglia had been activated. On the other hand, in the imNK cell administered group (Tg+imNK), a reduction in the activity of microglia was confirmed (Figure 8, red: IBA1, blue: cell nucleus (DAPI)). The above results confirmed that imNK cells can exhibit an excellent effect in preventing or treating dementia.

### Example 2.4. Confirmation of changes in genes in microglia in dementia mouse model according to administration of imNK cells

On day 40 of administering imNK cells to the mouse model of dementia in the same manner as above, the brains of the mice were extracted, and the brain cells were collected through a density gradient method using percoll. The collected brain cells were categorized and separated into microglia and non-microglia through MACS sorting. RNA was extracted from the separated microglia and non-microglia, and a transcriptome analysis was carried out.

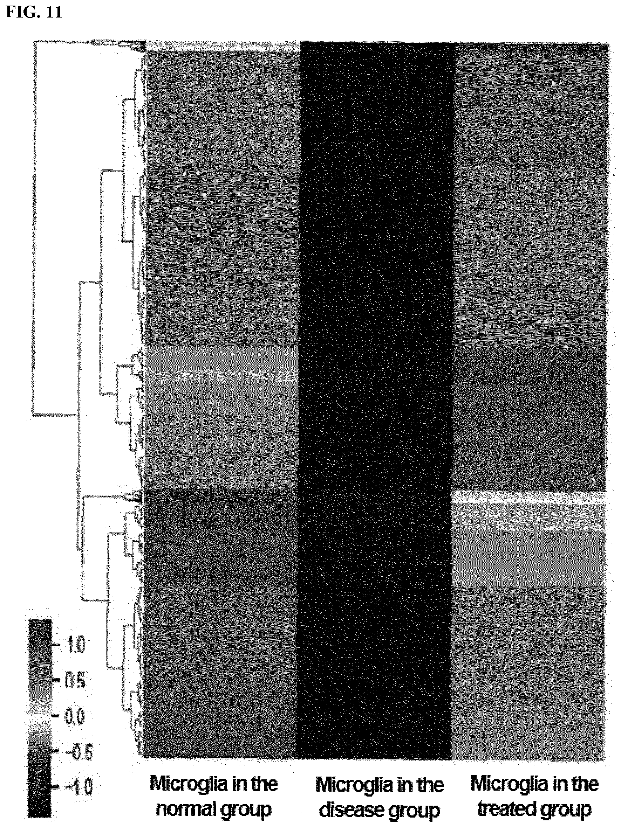

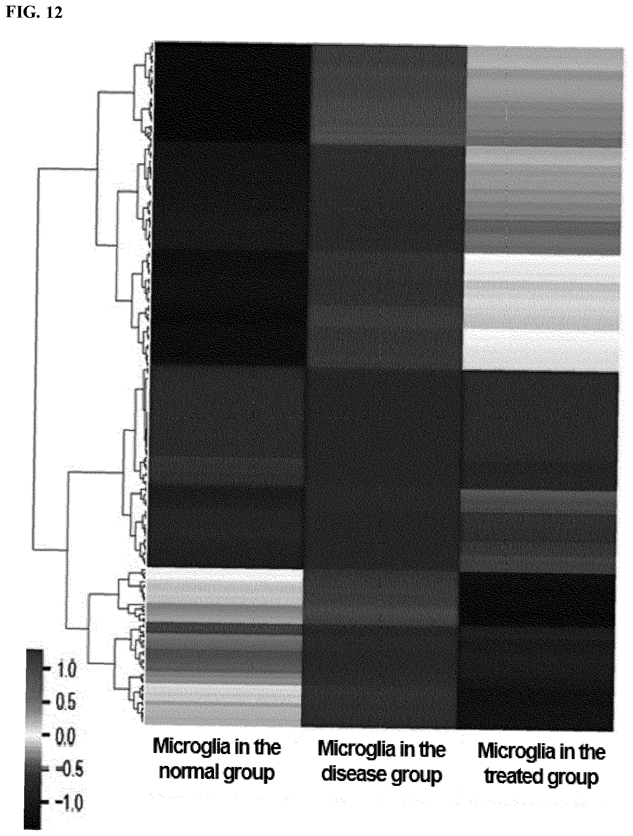

For a total of 23,282 genes, changes in the genes (6,836 in total) corresponding to the biological processes were confirmed. It was analyzed that 1,115 genes had decreased expression in the dementia model mouse group (sham, disease group) compared to the normal mouse group (normal group), but their expression was restored in the imNK cell-treated group (treated group) (Figure 11). It was further analyzed that 406 genes had increased expression in the dementia model mouse group (disease group), but decreased expression in the imNK cell-treated group (treated group) (Figure 12).

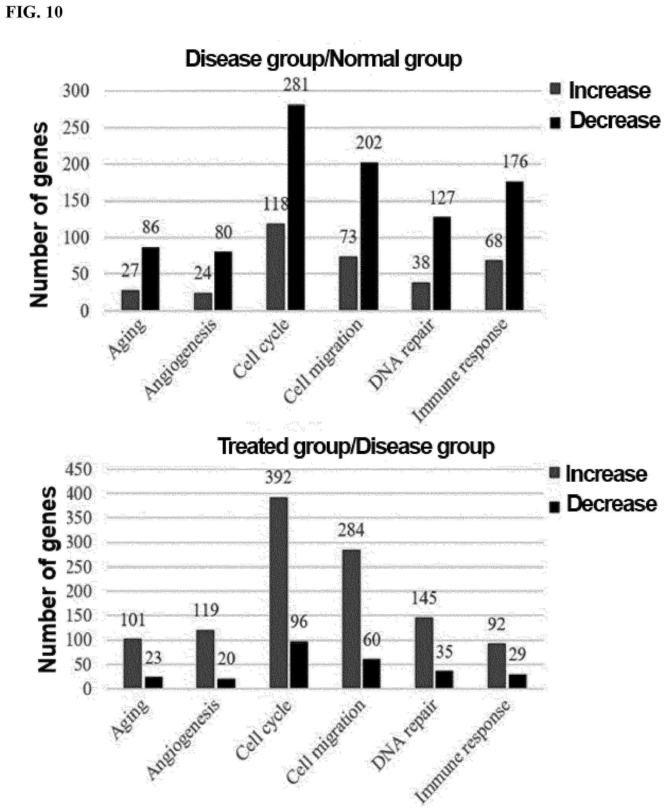

In addition, in the gene ontology corresponding to the biological process above, change patterns were examined in the genes corresponding to aging, angiogenesis, cell cycle, cell migration, DNA repair, and immune response. The results confirmed a tendency of reduced expression of genes involved in the corresponding gene ontology in the mouse model of dementia (sham, disease group) compared to the normal mouse group (normal group) (top of Figure 10). On the other hand, comparison between the imNK cell administered group (treated group) and the dementia-induced control group (sham, disease group) confirmed a tendency of an increase in the expression of genes involved in the gene ontology that had been reduced by dementia induction (bottom of Figure 10).

The above results confirmed that the development of dementia reduced the expression of the major genes associated with biological functions, and the administration of imNK cells was able to recover the expression of genes reduced by dementia.

### Example 2.4.1. Confirmation of changes in the genes associated with metabolic processes within microglia in dementia mouse model according to administration of imNK cells

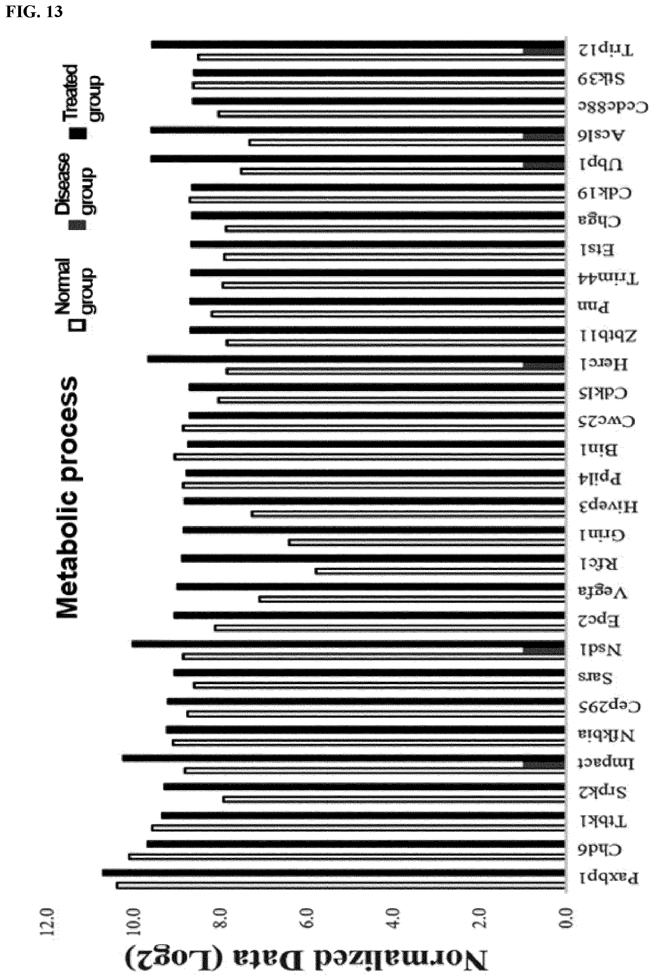

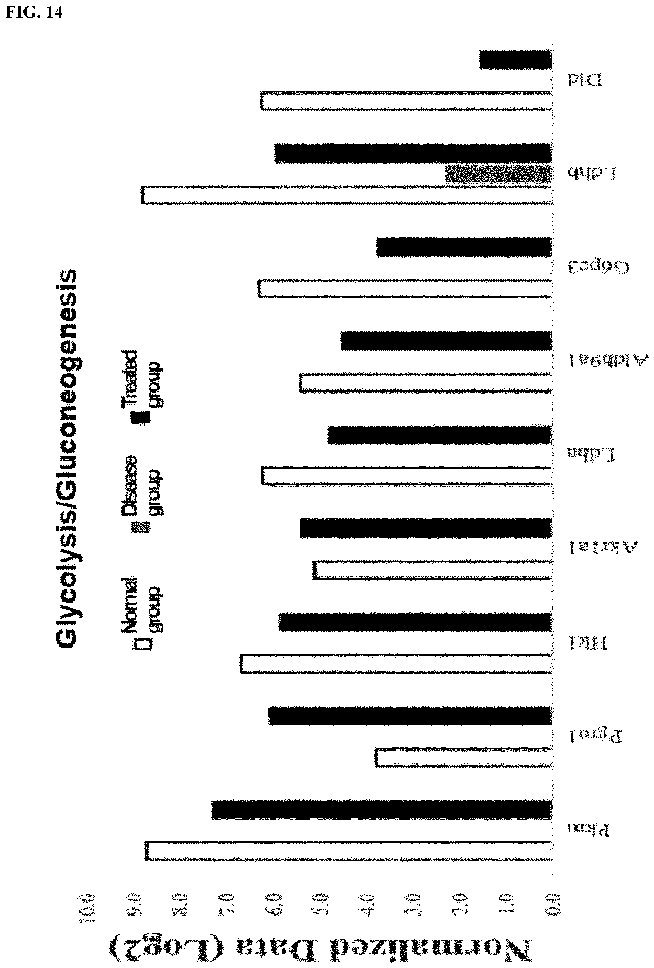

From the transcriptome analysis results of Example 2.4, it was identified that 2,370 genes in total were changed in relation to metabolic processes. Among these, it was identified that the expression of 1,694 genes was reduced by dementia induction (sham, disease group), but recovered by the administration of imNK cells (treated group). Among the genes changed, the patterns of change in the top 30 genes are shown in Figure 13. In addition, the analysis confirmed 9 genes corresponding to glycolysis/gluconeogenesis among the genes involved in metabolic processes, and the results thereof are shown in Figure 14.

### Example 2.4.2. Confirmation of changes in genes associated with immune responses within microglia in mouse model of dementia according to administration of imNK cells

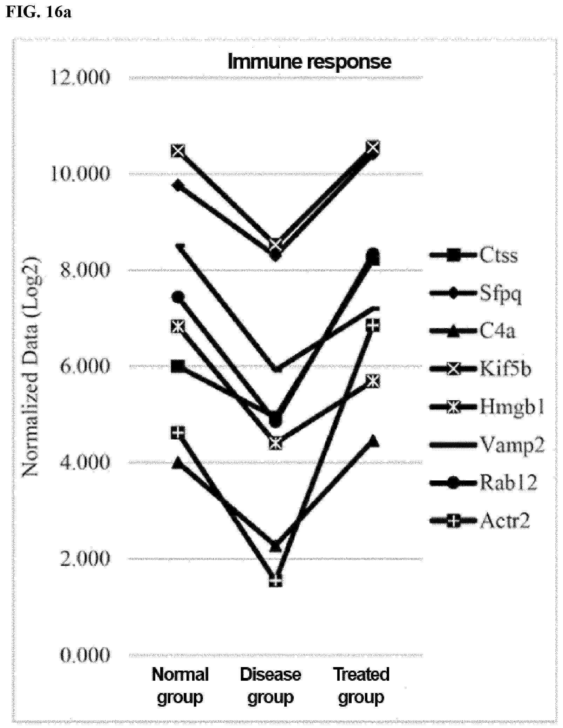

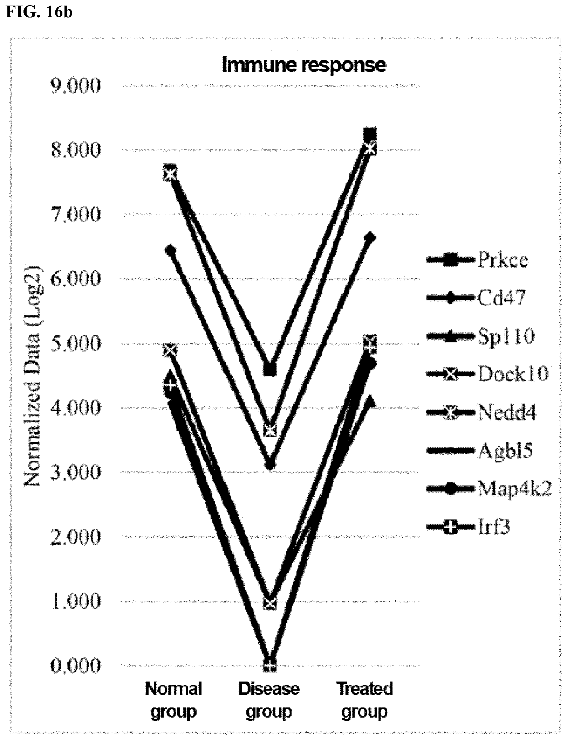

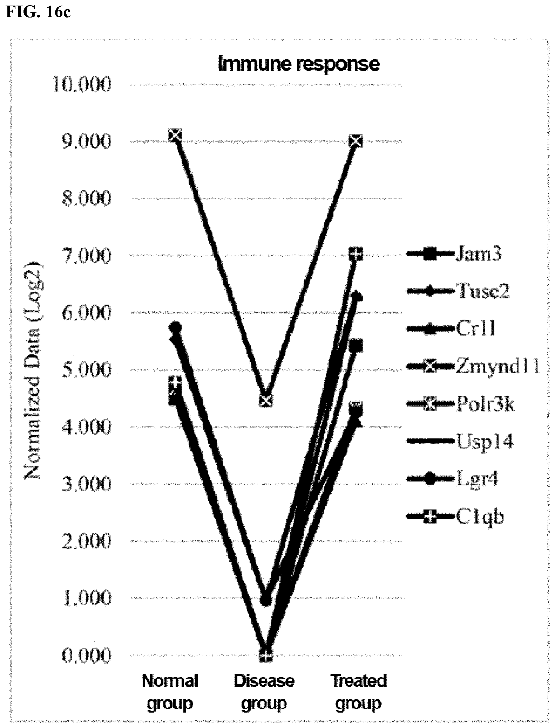

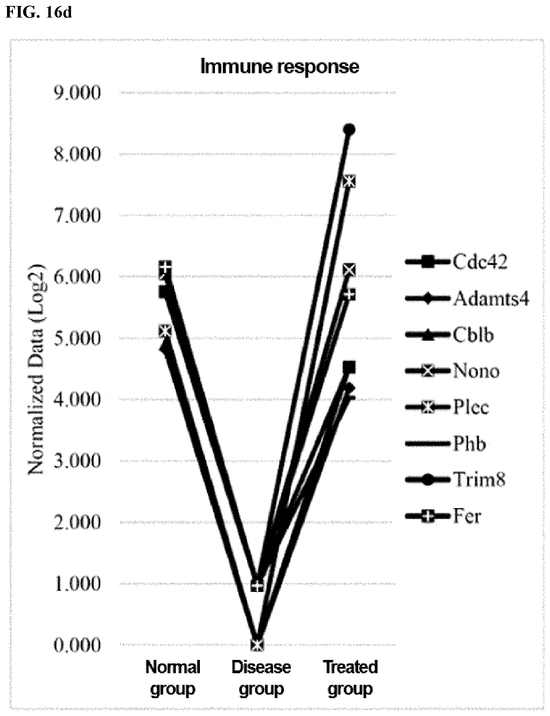

From the transcriptome analysis results of Example 2.4, it was identified that 178 genes in total were changed in relation to the immune responses. Among these, it was identified that the expression of 120 genes was reduced by dementia induction (sham, disease group), but recovered by the administration of imNK cells (treated group). Among the genes changed, the patterns of change in the top 30 genes are shown in Figures 16a to 16d.

### Example 2.4.3. Confirmation of changes in genes associated with neurogenesis within cells other than microglia in mouse model of dementia according to administration of imNK cells

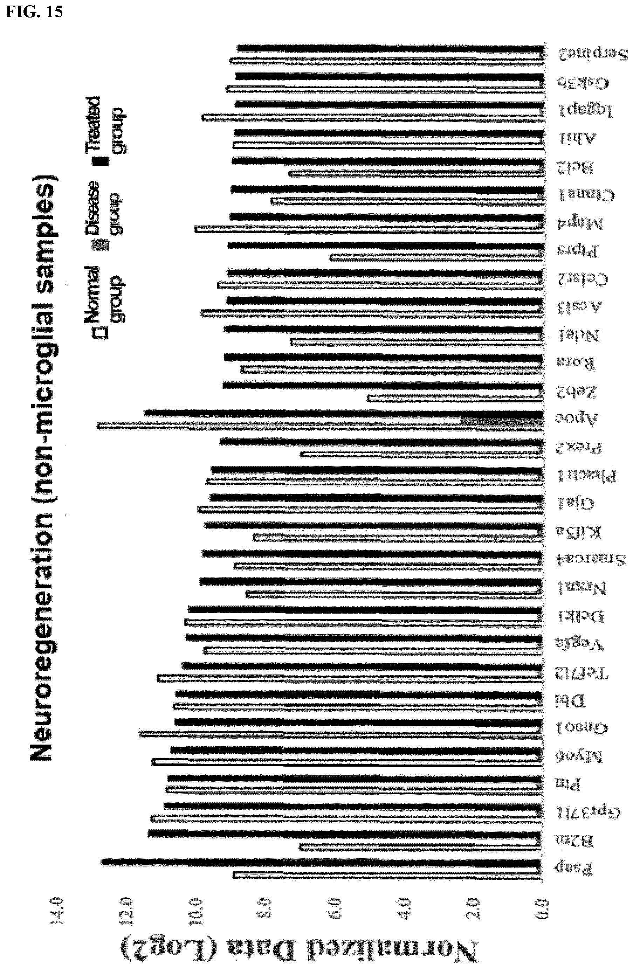

From the transcriptome analysis result in cells other than microglia in Example 2.4, it was identified that 226 genes were changed in relation to neurogenesis. Among these, it was identified that the expression of 172 genes was reduced by dementia induction (sham, disease group), but recovered by the administration of imNK cells (treated group). Among the genes changed, the patterns of change in the top 30 genes are shown in Figure 15.

### Example 2.4.4. Confirmation of changes in genes associated with autophagy within microglia in dementia mouse model according to administration of imNK cells

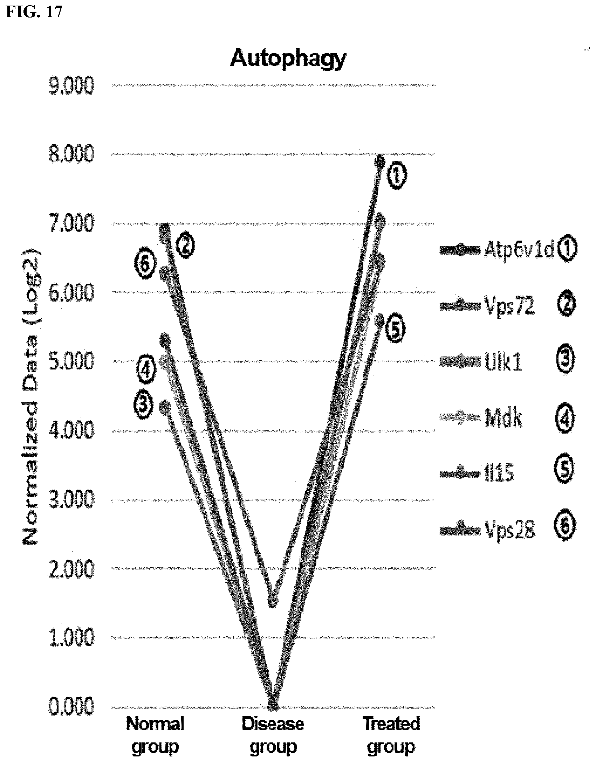

From the transcriptome analysis result in cells other than microglia in Example 2.4, the genes involved in autophagy were identified. Among these, it was identified that the expression of 97 genes was reduced by dementia induction (sham, disease group), but recovered by the administration of imNK (treated group). Among these genes, the results of identifying the patterns of changes in expression of Atp6v1d, Vps72, ulk1, Mdk, I115 and Vps28 are shown in Figure 17.

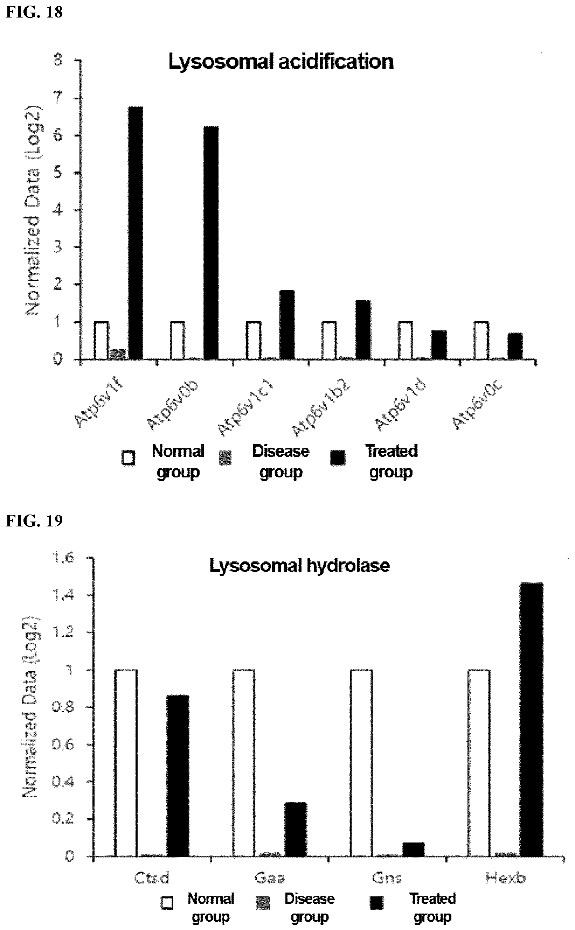

In addition, in relation to lysosomes, the patterns of changes in gene expression corresponding to lysosomal acidification or lysosomal hydrolase were identified, and the results thereof are shown in Figures 18 and 19.

### Example 3. Comparison of gene expressions in inactive and activated natural killer cells

### Example 3.1. Induction of activation of natural killer cells derived from human peripheral blood mononuclear cells

After mixing human peripheral blood mononuclear cell-derived natural killer cells (PBMC) with the medium at a ratio of 1:1, the mixture was loaded into a tube containing ficoll (Ficoll:blood:medium = 1:1:1). The tube loaded with the mixture was centrifuged at 400 g for 30 minutes and 30 seconds (ACC:4, DEC:0) to collect a white blood cell layer (the second layer). The cells of the collected layer were cultured in a 37°C, 5% CO₂ incubator using a natural killer cell medium including 2% human AB serum, 1% L-Glutamine, 50 ng/ml of IL-15, 3 ng/ml of IL-12, and 30 ng/ml of IL-18. On day 5 of the culture, the cells were transferred from the T-25 flask to the T-75 flask, and then treated to contain 1% L-Glutamine, 2% human AB serum, 500 IU/ml of IL-2, and 1 ng/ml of IL-15 per 10 ml of medium to be cultured in a 37°C, 5% CO₂ incubator. On day 14 of the culture, the cells were collected and used as activated PBMC-derived natural killer cells (hereinafter referred to as PBNK or PBNK cells).

### Example 3.2. Comparative analysis of gene expressions in inactive and activated natural killer cells

PBNK cultured in the same manner as in Example 3.1 was collected on day 0 of the culture (inactive PBNK) and day 14 of the culture (activated PBNK) to extract RNA. The extracted RNA was used to carry out mRNA sequencing using QuantSeq 3' mRNA-Seq to analyze gene expressions. From the results of identifying the change in gene expression levels using ExDEGA, genes with a fold change of 2 or more and normalized data (Log2) showing an expression level of 4 or more were selected, and the distribution of gene ontology corresponding to the biological processes was identified. The tendency of genes changed in each gene ontology is as follows:
Aging (upstream: 30, 10.49%), angiogenesis (upstream: 24, 7.55%), apoptotic process (upstream: 135, 14.45%), cell cycle (upstream: 332, 24.76%), cell death (upstream: 145, 13.07%), cell differentiation (upstream: 323, 8.42%), cell migration (upstream: 83, 9.13%), DNA repair (upstream: 128, 25.75%), extracellular matrix (upstream: 30, 5.20%), immune response (upstream: 190, 9.97%), inflammatory response (upstream: 56, 9.98%), neurogenesis (upstream: 142, 8.22%), RNA splicing (upstream: 54, 12.95%), and secretion (upstream: 114, 9.95%).

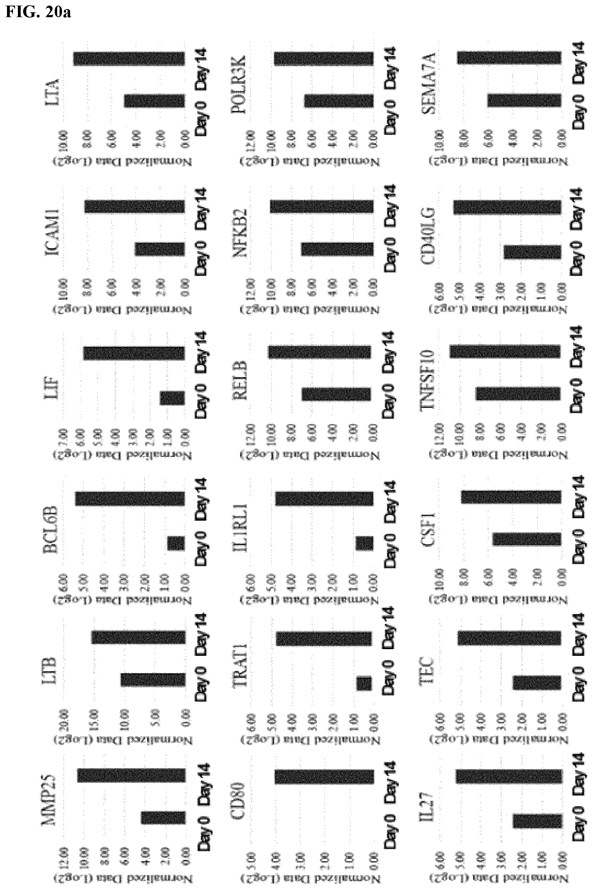

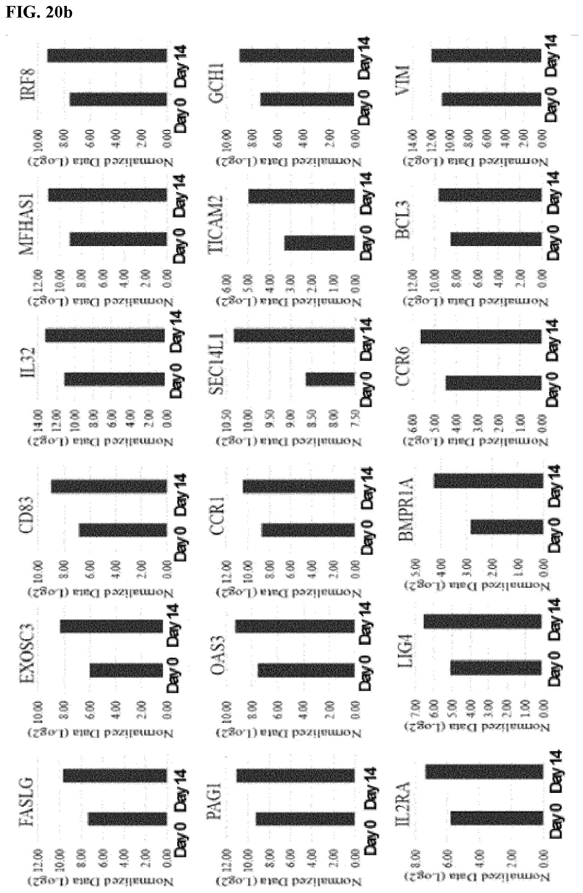

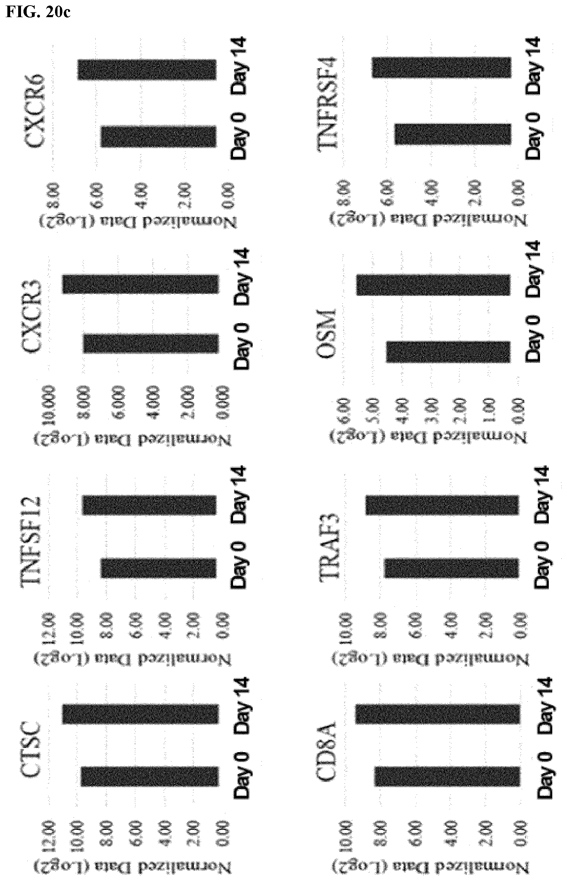

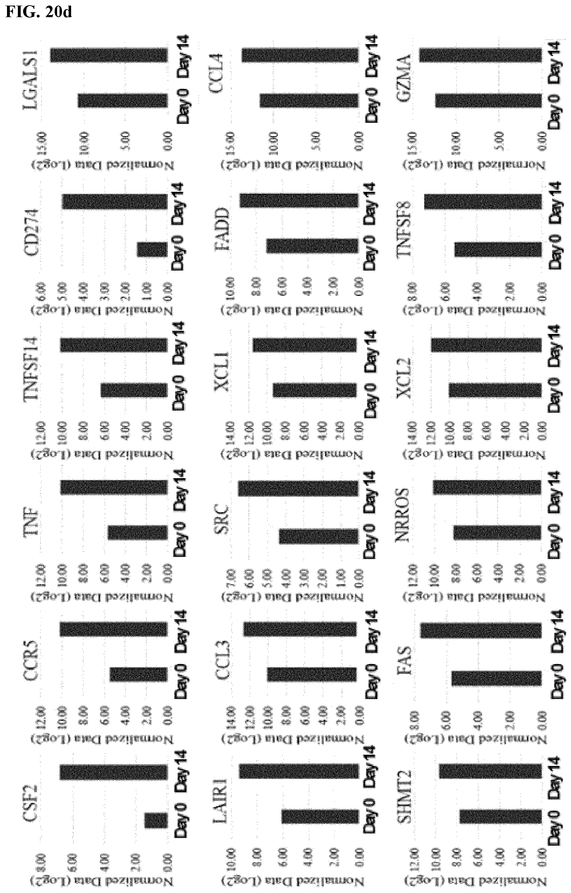

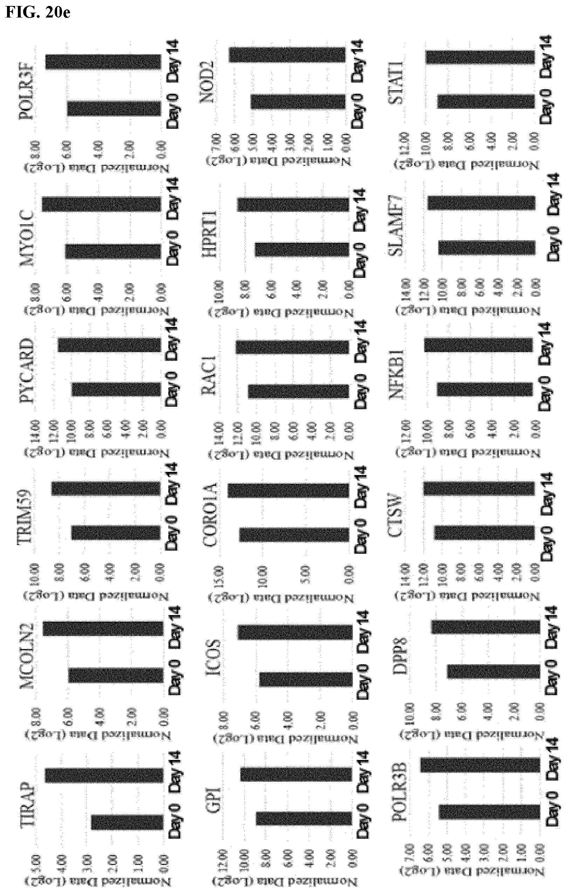

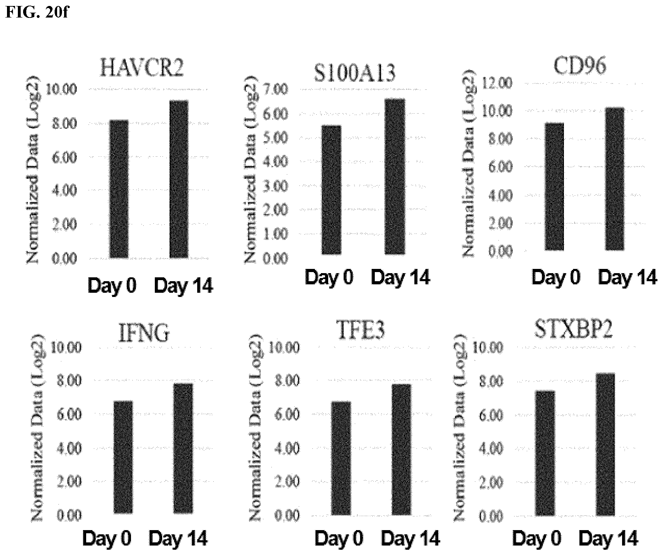

In particular, from the genes relating to gene ontology, it was identified that the expression of 190 genes increased among the genes related to the immune responses in activated PBNK compared to inactivated PBNK. Among these genes, the expression of 86 genes related to the effector function and immune cell activation was comparatively analyzed, and the results thereof are shown in Figures 20a to 20f.

### Example 4. Comparison of gene expressions in activated PBNK and activated induced pluripotent stem cell-derived natural killer cells

### Example 4.1. Method for producing natural killer cells from human induced pluripotent stem cells

The CMC-009 human induced pluripotent stem cell (iPSC) cell line used in the present examples was obtained from the National Stem Cell Bank at the National Center for Stem Cell and Regenerative Medicine. The cell line was cultured in a StemFit Basic04 culture medium (Ajinomoto, AJBASIC04) in a cell culture container applied with iMatrix-511 (Nippi, 892012), which is an extracellular matrix.

Specifically, it was cultured with a serum-free stem cell culture medium, Essential 8^{™} (Gibco, 1517001) further containing 2 uM CHIR-99021 (Peprotech, 2520691), 80 ng/ml of BMP4 (R&D Systems, 314-BP/CF), and 80 ng/ml of VEGF (R&D Systems, 293-VE/CF). After 2 days, the culture medium was removed, and the cells were cultured in a serum-free stem cell culture medium, Essential 6^{™} (Gibco, 1516401) further containing 2 uM SB-431542 (Peprotech, 3014193), 50 ng/ml of SCF (R&D Systems, 255-S/CF), and 80 ng/ml of VEGF to induce differentiation into mesoderm cells, and then differentiation into hematopoietic stem cells was additionally induced. After 8 days, the culture medium was removed, and differentiation into natural killer cells was induced.

### Example 4.2. Induction of activation of EiNK

Natural killer cells derived from human induced pluripotent stem cells (hereinafter referred to as EiNK or EiNK cells) obtained using the method of Example 4.1 were further cultured by adding complete media (RPMI1640 including 1% L-Glutamine, 15% human AB serum, 1% penicillin/streptomycin, 50 IU/ml IL-2, 10 ng/ml IL-15, and 5 ng/ml sodium selenite). On day 21 of the culture, the cells were collected to be used as activated EiNK.

### Example 4.3. Analysis of gene expressions in activated PBNK and activated EiNK

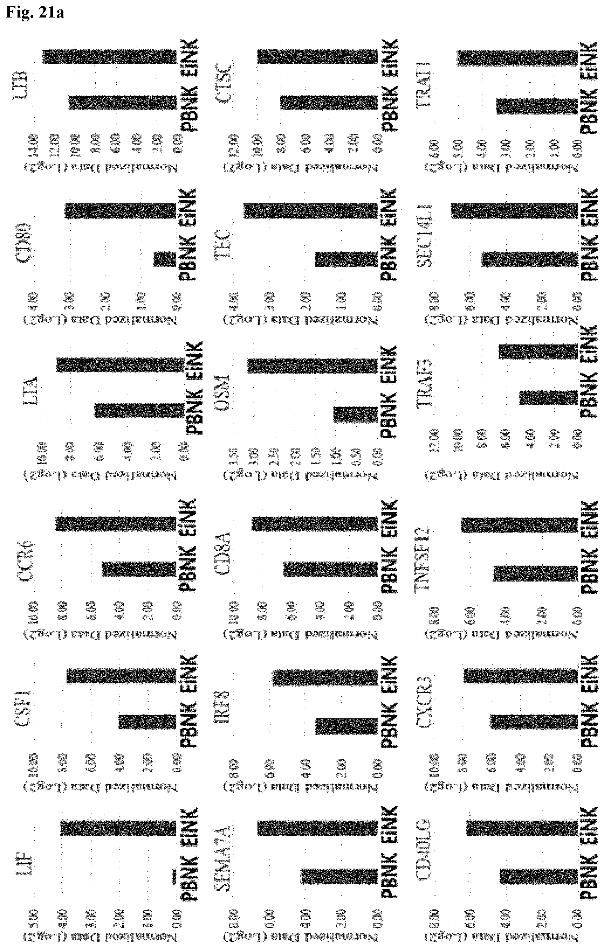

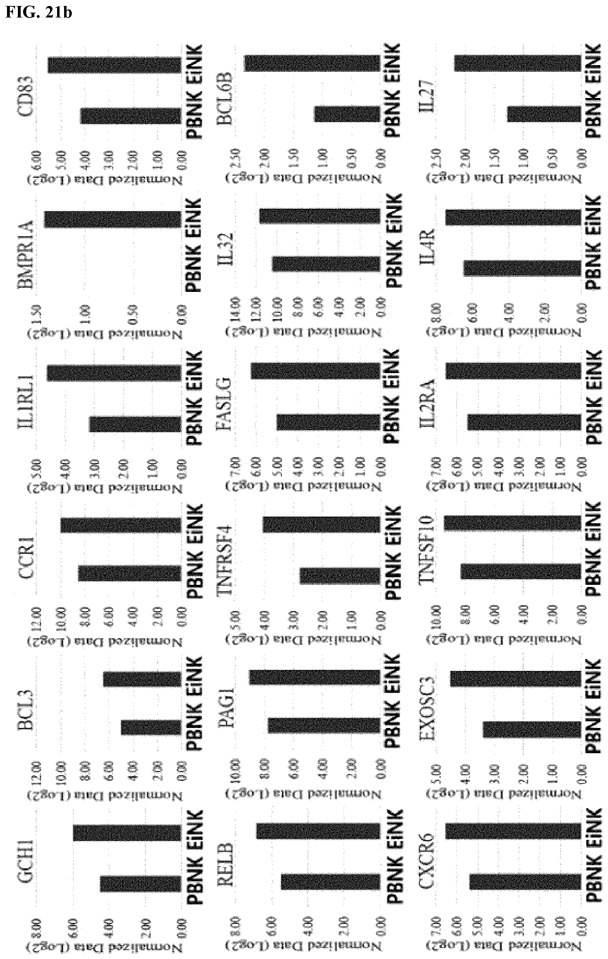

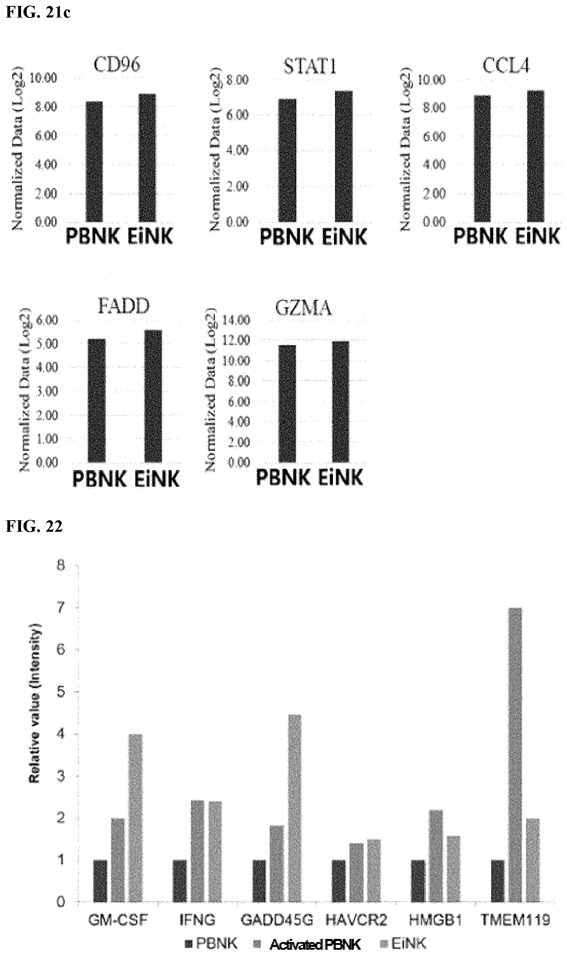

PBNK activated using the method of Example 3.1 and EiNK activated using the method in Example 4.2 were used to extract RNA from the cells activated for 14 days. The extracted RNA was used to carry out mRNA sequencing using QuantSeq 3' mRNA-Seq to confirm gene expressions. From the results of identifying the change in the gene expression levels using ExDEGA, 41 genes with a fold change of 2 or more were selected, which are shown in Figures 21a to 21c.

The 41 genes are genes related to immune responses, and this result suggests that human EiNK according to the present disclosure can treat Alzheimer's disease.

In addition, the expression of CSF2 (GM-CSF), IFNG, GADD45G, Zbtb32, P2RY14, IL12RB1, CD55, SEMA6D, GZMB, HAVCR2, CXCR6, HMGB1, IL2, IL10, and TMEM119, which are genes observed to have increased expression in imNK through the transcriptome analysis in the three types of NK cells, was confirmed. As a result, as shown in Figure 22, the gene expressions of GM-CSF, IFNG, GADD45G, HAVCR2, HNGB1, and TNEN119 increased not only in activated PBNK compared to inactive PBNK but also in activated EiNK.

The above results confirmed that the expression of the 6 types of genes that are important in immune regulation increased not only in mouse-derived natural killer cells but also in human-derived natural killer cells (PBNK and EiNK). In addition, these results suggest that human-derived natural killer cells according to the present disclosure may also normalize the metabolism and the function of microglia, which play an important role in Alzheimer's disease or dementia, in a similar manner to the mouse-derived natural killer cells.

## Claims

1. A pharmaceutical composition for preventing or treating a degenerative brain disease comprising natural killer cells as an active ingredient.

2. The pharmaceutical composition for preventing or treating a degenerating brain disease according to claim 1, wherein the natural killer cells overexpress any one specific gene selected from the group consisting of GM-CSF, IFN-gamma, Gadd45g, zbtb32, P2RY14, TREM119, IL12RB1, CD55, Sema6D, Gzmb/c/d/e/f, Havcr2, CXCR6, HMGB1, IL-2, IL-10 and combinations thereof.

3. The pharmaceutical composition for preventing or treating a degenerating brain disease according to claim 2, wherein an expression of the gene is increased by two or more times compared to natural killer cells present in an individual.

4. The pharmaceutical composition for preventing or treating a degenerating brain disease according to claim 2, wherein an expression of the gene is increased when natural killer cells obtained from an individual are cultured for 1 to 28 days.

5. The pharmaceutical composition for preventing or treating a degenerating brain disease according to claim 4, wherein the natural killer cells are cultured in the presence of any one selected from the group consisting of IL-15, IL-2, and combinations thereof.

6. The pharmaceutical composition for preventing or treating a degenerating brain disease according to claim 1, wherein the natural killer cells are obtained from any one selected from the group consisting of placental tissue, placental perfusate, umbilical cord blood, placental blood, peripheral blood, spleen, liver, and induced pluripotent stem cells (iPSC).

7. The pharmaceutical composition for preventing or treating a degenerating brain disease according to claim 1, wherein the natural killer cells are activated.

8. The pharmaceutical composition for preventing or treating a degenerating brain disease according to claim 7, wherein the activated natural killer cells increase the secretion of INF-gamma and/or GM-CSF by two or more times compared to natural killer cells present in an individual.

9. The pharmaceutical composition for preventing or treating a degenerating brain disease according to claim 1, wherein the degenerative brain disease is selected from the group consisting of Alzheimer's disease, Parkinson's disease, Huntington's disease, mild cognitive impairment, cerebral amyloid angiopathy, Down syndrome, amyloid stroke, systemic amyloidosis, amyloidosis-Dutch type, Niemann-Pick disease, senile dementia, amyotrophic lateral sclerosis, spinocerebellar atrophy, Tourette's syndrome, Friedrich's ataxia, Machado-Joseph disease, Lewy body dementia, vascular dementia, dystonia, progressive supranuclear palsy, and frontotemporal dementia.

10. Use of the pharmaceutical composition of claim 1 for preventing or treating a degenerative brain disease.

11. A method of treating a degenerative brain disease comprising administering the pharmaceutical composition of claim 1 to a subject.
